# EUROPEAN PATENT SPECIFICATION

(11) **EP 4 015 033 B1**
(45) Date of publication and mention of the grant of the patent: **16.07.2025**
(21) Application number: 20216246.7
(22) Date of filing: 21.12.2020
(51) Int. Cl.: A61N 1/36, A61N 1/375

(54) **FLEXIBLE ELECTRODE CARRIER**
FLEXIBLER ELEKTRODENTRÄGER
SUPPORT D'ÉLECTRODE SOUPLE

(43) Date of publication of application: 22.06.2022
(73) Proprietor: INBRAIN Neuroelectronics SL, 08028 Barcelona (ES)
(72) Inventor: Bakker, Bert, Wijk en Aalburg (NL); Decré, Michel, 5656 AE Eindhoven (NL); Garrido Ariza, José Antonio, 08960 Sant Just Sesvern (Barcelona) (ES)
(74) Representative: DTS Patent- und Rechtsanwälte PartmbB

(56) References cited:
- EP-A1- 3 702 327
- DE-A1- 102014 009 890
- DE-B3- 102006 006 263
- US-A- 5 876 443
- US-A1- 2007 288 076
- US-A1- 2020 222 694
- US-B1- 6 374 143
- NICHOLAS V. APOLLO ET AL: "Soft, Flexible Freestanding Neural Stimulation and Recording Electrodes Fabricated from Reduced Graphene Oxide", ADVANCED FUNCTIONAL MATERIALS, vol. 25, no. 23, 1 June 2015 (2015-06-01), DE, pages 3551 - 3559, XP055744108, ISSN: 1616-301X, DOI: 10.1002/adfm.201500110
- MATTIA BRAMINI ET AL: "Interfacing Graphene-Based Materials With Neural Cells", ARXIV.ORG, CORNELL UNIVERSITY LIBRARY, 201 OLIN LIBRARY CORNELL UNIVERSITY ITHACA, NY 14853, 20 March 2019 (2019-03-20), XP081155763, DOI: 10.3389/FNSYS.2018.00012
- NICHOLAS V APOLLO ET AL: "Soft, Flexible Freestanding Neural Stimulation and Recording Electrodes Fabricated from Reduced Graphene Oxide", ADVANCED FUNCTIONAL MATERIALS, WILEY - V C H VERLAG GMBH & CO. KGAA, DE, vol. 25, no. 23, 4 May 2015 (2015-05-04), pages 3551 - 3559, XP072291277, ISSN: 1616-301X, DOI: 10.1002/ADFM.201500110

## Description

The present invention relates to a flexible electrode carrier, especially for brain stimulation, in particular for Cortical and/or Deep Brain Stimulation.

In practice it is known that Cortical and/or Deep Brain Stimulation is a method for the diagnosis and therapy of neurodegenerative diseases such as inter alia the Parkinson's disease, epilepsy, and chronic pain. In this respect, electrical stimulation by means of leads which were implanted into brain areas or regions like the subthalamic nucleus and/or the globus pallidus internus can e.g. alleviate the tremor symptoms of a patient suffering from a drug-resistant Parkinson's disease. Further, the signals from a brain region or area at which the leads were implanted can be recorded and the state of the brain tissue can be determined using impedance or physiological measurements.

Typically, the Deep Brain Stimulation (DBS) is performed with two leads each implanted into one hemisphere of the brain. Each lead can comprise an array of e.g. 4 or 8 electrodes arranged in a ring around the core body of the lead, wherein the number and shape of the electrodes as well as the specific arrangement of the electrodes on the lead determines the electrical stimulation field or wave which affects the brain tissue.

Bramini M, Alberini G, Colombo E, Chiacchiaretta M, DiFrancesco ML, Maya-Vetencourt JF, Maragliano L, Benfenati F, Cesca F. Interfacing Graphene-Based Materials With Neural Cells. Front Syst Neurosci. 2018 Apr 11;12:12. doi: 10.3389/fnsys.2018.00012. PMID: 29695956; PMCID: PMC5904258 discloses different applications for graphene-based materials, e.g. in neuroscience, inter alia for neural stimulation and recording. The document teaches a graphene-based electrode carrier for *in vivo* recording, said electrode carrier comprising an electrode array arranged on a flexible substrate.

Apollo, N.V., Maturana, M.I., Tong, W., Nayagam, D.A.X., Shivdasani, M.N., Foroughi, J., Wallace, G.G., Prawer, S., Ibbotson, M.R. and Garrett, D.J. (2015), Soft, Flexible Freestanding Neural Stimulation and Recording Electrodes Fabricated from Reduced Graphene Oxide. Adv. Funct. Mater., 25: 3551-3559 discloses a "brush-like" electrode fabricated from wet-spun rGO fibers, the electrode having an expanded, thus less dense, end, which is softer than the original fibers. A Parylene-C covering is provided, acting as biocompatible insulator and elasticity enhancer.

In Daria Nesterovich Anderson et al, Journal of Neural Engineering 15, 2018, an optimized programming algorithm for cylindrical and directional deep brain stimulation leads is presented, since programming becomes especially important if the DBS lead does not precisely hit its targeted brain area because stimulation that spreads away from the intended target region or area may lead to psychiatric, non-motor, and motor side effects. Even with state-of-the-art surgical techniques, it is typical to see deviation from the target area by 2 mm to 4 mm during surgery, especially after the first lead in bilateral DBS surgery has been implanted. In general, if the distance from the target is greater than 3 mm, the lead or the electrodes do not reach any portion of the area physiologically defined as optimal. Such placement errors have been reported to occur in over 40% of implantations. The prevalence of lead placement deviation has motivated the creation of directional DBS leads. With a greater number of smaller electrodes, directional leads can generate asymmetric stimulation profiles and steer stimulation back toward a missed target. However, the increasing complexity of directional leads compounds the clinical difficulty and time burden associated with their programming.

Therefore, it is an object of the present invention to advantageously further develop a flexible electrode carrier of the aforementioned kind, in particular in such a way that more than one targeted brain area can be reached better with only one flexible electrode carrier and that the stimulation field can be adjusted more accurately in relation to the targeted brain area.

The aforesaid object with respect to the flexible electrode carrier is achieved by the features of claim 1. Advantageous configurations of the invention regarding the flexible electrode carrier are described in dependent claims 2 to 14.

According to the present invention, a flexible electrode carrier for brain stimulation, in particular for Cortical Brain Stimulation and/or Deep Brain Stimulation, having a proximal end and a distal end, wherein the flexible electrode carrier comprises at least two zones each comprising a plurality of electrodes, wherein the material of which the electrodes are made is reduced-graphene oxide, wherein the plurality of electrodes in each of the at least two zones comprise at least one type of electrodes, and wherein the types of electrodes are each arranged in an individual pattern within the zones.

The basic idea of the present invention is to stimulate different areas of the brain tissue as well as recording signals from the same, wherein these targets do not have to be arranged or situated in a straight line, since the flexible electrode carrier or lead can be adapted to perfectly fit to the at least two different brain areas by aligning the at least two zones to at least two brain areas. Moreover, the respective plurality of electrodes within each zone may be different so that possible anatomical or functional differences between the brain areas to be stimulated and/or recorded can be considered, wherein the respective plurality of electrodes within each zone can further be heterogeneous, i.e. different types of electrodes can be provided within each zone to be able to precisely adapt the generated stimulation field and or the recording resolution to the adjacent brain tissue and its physiological conditions. In other words: By having more active regions on the same electrode carrier (also possible to call this a lead), whereas an active region shall be understood as region of at least two (grouped) electrode, further having electrodes with e.g. different function(s) or electrodes with varying anatomical shape, several regions to be stimulated can be addressed concurrently with one lead. This reduces the invasiveness of the therapy.

Additionally, it can help to add new functionality and offers reduced system complexity and scaling. Also the effectiveness of the therapy increase and new therapies can be developed, as new features offer new ways of therapy.

Further, also dynamics of the brain circuits can be targeted and subject of the treatment. There could be instances also where the effect of stimulation provided at brain region A, is best observed in a brain activity from brain region B, or that the best region B is determined by evoked potentials triggered in region C, while therapeutic stimulation region is A.

The degree of flexibility of the flexible electrode carrier is chosen in such a way that, on the one hand, an adaptation of the shape of the flexible electrode carrier is possible, on the other hand the degree of flexibility, but on the other hand, the degree of flexibility also ensures that the required shape into which the flexible electrode carrier is brought to reach the desired brain areas to be stimulated is substantially maintained after insertion.

The flexible electrode carrier can be in the form of a lead or a lead paddle or a patch or any other suitable structure.

The material for the flexible electrode can be e.g. polyurethanes and silicones or the like. Any biocompatible polymeric material can be used.

The distal end of a flexible electrode carrier is defined as the end that is leading during insertion, while the proximal end of the flexible electrode carrier is closer to either the opening in the cranium and/or the pulse generator.

The at least two zones can be arranged along the flexible electrode carrier either directly adjacent or spaced from each other, preferably with one of the zones at the distal end.

It is possible that the flexible electrode carrier comprises at least 32 stimulation electrodes to be able to stimulate a respective brain area with sufficient precision, and 32 recording electrodes to achieve a sufficiently high resolution. Also other numbers are possible. Choosing a higher number of electrodes (especially higher than 4 or 8 electrodes as now commonly used) is desirable, as electrode sizes of 4- or 8- groups have limitations in the resolution of functionally relevant brain signals, and the resolution of addressable relevant brain regions will be enhanced if a higher number of stimulation electrodes is used.

In a possible embodiment, the flexible electrode carrier is at least substantially elongated.

An elongated electrode carrier allows for reaching different, spaced areas of the brain which might be particularly useful to further understand the effects of stimulation also on neighboring brain areas and possibly pathologic brain circuitry. Furthermore, it is conceivable that one zone of the flexible electrode carrier generates a stimulation field by means of the electrodes, while the electrodes of the other zones record the signals of other brain areas, which signals, among other things, can be regarded as a direct consequence of the effect of the stimulation field. It should be understood that the stimulation field is generated by an electrical impulse generator, which controls the flexible electrode carrier.

A further advantage of the elongated extension of the flexible electrode carrier is that only a small opening or through hole in the cranium needs to be prepared for insertion and placement of the flexible electrode carrier.

In a further possible embodiment, the plurality of electrodes in each of the at least two zones comprise at least one type of electrodes.

This means that either a certain type of electrodes is provided in a zone or that several types of electrodes, preferably two types of electrodes, are provided within a zone. Subsequently, different stimulation fields can be generated by means of a zone having more than one type of electrodes. Preferably, the different types of electrodes can be controlled separately. In case the different types are controlled simultaneously to generate a stimulation field, it can be assumed that the respective fields superpose each other. Furthermore, it can also be provided that, for example, with two types of electrodes, one type generates a stimulation field while the other type records the emitted signals of the affected brain area. It is therefore advantageous if a zone is configured in such a way that the respective types of electrodes can be individually connected and controlled.

In another possible embodiment, the type of an electrode is at least one of the group of size, shape, and material.

The types of electrodes of an inserted flexible electrode carrier must be able to meet the requirements of the adjacent brain area, in particular to avoid damaging it. Hence, each type of electrode has a specific characteristic comprising the size, the shape and the material of which the electrode is made.

The shape can be e.g. ring-shaped or rectangular or the like, in order to be able to stimulate the corresponding brain area or region in the best possible way without affecting or even impairing adjacent areas.

Different sizes of electrodes may be necessary to generate a stimulation field corresponding to the size of the targeted brain area.

Since the electrodes are controlled electronically or are applied with a certain voltage, it is necessary that the material corresponds to the periodic electrical impulses from the pulse generator, i.e. that it withstands these impulses over a predetermined duration and is not prematurely worn out or, degraded, or corroded. It is particularly important that if an electrode is accidentally overloaded or if there is a material defect, and parts of the electrode become detached, these parts are not toxic to the surrounding brain tissue.

Generally speaking, several types of electrodes can be used on the flexible electrode carrier. The types of electrodes can differ in at least one the parameters size, shape, layout, material, and whether they are used for stimulation or recording or both.

In a further possible embodiment, the material of which the electrodes are made is reduced-graphene oxide or platinum-iridium alloy.

Reduced-graphene oxide is characterized by its high durability, superior conductivity, and resistance to high level charges, which is why it is particularly suitable as material for the electrodes. One thing to consider is that reduced-graphene oxide is very brittle, which is why it is advisable to cover reduced-graphene oxide electrodes with a protective layer to particularly protect against mechanical impacts.

However, there can be sections or parts of the electrodes, which are not covered.

Also, it can be foreseen that the electrodes are not covered with a protective layer.

With no protective layer or a partial protective layer, the electrodes can directly contact brain tissue and cerebrospinal fluid can enter into the electrode.

Alternatively, a platinum-iridium alloy, iridium oxide, activated iridium, platinum, or any other commonly used electrode metal/material can be used where the advantageous properties of reduced-graphene oxide may not be essential.

In yet another possible embodiment, the types of electrodes are each arranged in an individual pattern within the zones.

In order to correspond to the brain area to be stimulated, it is also advantageous if the different types of electrodes within a zone are arranged in type-related patterns, whereby e.g. two electrode types of different size can stimulate two portions of the targeted brain area differently, i.e. with two different intensities or with different activating functions. An activating function is a mathematical formalism that is used to approximate the influence of an extracellular field on an axon or neuron. It was developed by Frank Rattay and is a useful tool to approximate the influence of functional electrical stimulation (FES) or neuromodulation techniques on target neurons. It points out locations of high hyperpolarization and depolarization caused by the electrical field acting upon the nerve fiber. As a rule of thumb, the activating function is proportional to the second-order spatial derivative of the extracellular potential along the axon.

Additionally or alternatively, the flexible electrode carrier is widened at its distal end. Widening can be achieved by expanding the geometry, increasing the diameter, a change of shape or the like.

If the flexible electrode carrier is to be used for Cortical Brain Stimulation, it is advantageous or even required that the electrodes are arranged in a defined plane to cover are predetermined cortical area. Thus, widening of the distal end means that preferably some kind of pad is formed at the distal end in which the electrodes are arranged. Flexibility is also required for this pad so that the pad can adapt to the local curvature of the cortical are to be stimulated or recorded. Furthermore, the flexibility also facilitates that despite the widening at the distal end, the flexible electrode carrier can still be inserted through a narrow opening in the cranium, e.g. by wrapping or rolling the pad for insertion.

Alternatively, the widened distal end can also be formed in circular shape or closed contour, wherein the electrodes can be arranged along the circulation and/or inside the inner circular portion.

In a further possible embodiment, the electrodes of one zone are embedded in a thin film substrate.

A thin film is a layer of material ranging from fractions of a nanometer (monolayer) to several micrometers in thickness. The controlled synthesis of materials as thin films (a process referred to as deposition) is a fundamental step in many applications. The act of applying a thin film to a surface is thin film deposition - any technique for depositing a thin film of material onto a substrate or onto previously deposited layers. "Thin" is a relative term, but most deposition techniques control layer thickness within a few tens of nanometers. Thin film preparation techniques can be separated into non-vapor and vapor techniques. In particular, atomic layer deposition, PVD, CVD are aiming for atomic control, while wet spin-coating, dip-coating, (electro-)spray typically deposit microns to tens of microns in wet-phase, leaving final layers from tens of nanometers to several microns, depending on dry residue and solvent content.

Thin film processing offers design options that are unavailable to current, commonly used bulk metal electrodes. Such electrodes are provided by currently by the manufacturers and the only offer not more than 3 rectangular segments in the 1-1-3-3 or 1-3-3-1 segmented leads in DBS.

Besides, thin film processing offers more options to combine different materials by using masking, lift-off, and photoresist lithography, or additive techniques.

Within the layers forming the thin film substrate electrically conductive connections to the electrodes are provided so that the electrodes can be electrically controlled.

The thin film substrate comprising a plurality of electrodes of at least one type is wrapped around or applied to the respective zone of the flexible electrode carrier.

The advantage of this is that the actual carrier and the electrodes including the electrical connections can be manufactured separately which facilitates the assembly of a flexible electrode carrier.

In the following, further advantages and embodiments of the present invention are described in conjunction with the attached drawings. Thereby, the expression "left", "right", "below", and "above" are referred to the drawings in an orientation of the drawings which allows the normal reading of the reference signs. The drawings should not necessarily represent the forms of execution to scale. Rather, the drawings, where useful for explanation, are executed in schematic and/or slightly distorted form. The invention's features revealed in the description, in the drawings, and in the claims may be essential for any continuation of the invention, either individually or in any combination. The general idea of the invention is not limited to the exact form or detail of the preferred embodiments shown and described below or to a subject-matter which would be limited in comparison to the subject-matter of the claims. For the sake of simplicity, identical or similar parts or parts with identical or similar functions are hereinafter referred to by the same reference signs.

In the drawings:
- **Fig. 1**: a first embodiment of a flexible electrode carrier having two zones according to the present invention;
- **Fig. 2**: a second embodiment of a flexible electrode carrier having three zones according to the present invention;
- **Fig. 3**: an embodiment of a zone at a distal end of a flexible electrode carrier according to the present invention;
- **Fig. 4**: four embodiments of different zones of a flexible electrode carrier according to the present invention;
- **Fig. 5**: a first embodiment of a widened distal end of a flexible electrode carrier according to the present invention;
- **Fig. 6**: a second embodiment of a widened distal end of a flexible electrode carrier according to the present invention; and
- **Fig. 7**: a third embodiment of a widened distal end of a flexible electrode carrier according to the present invention.

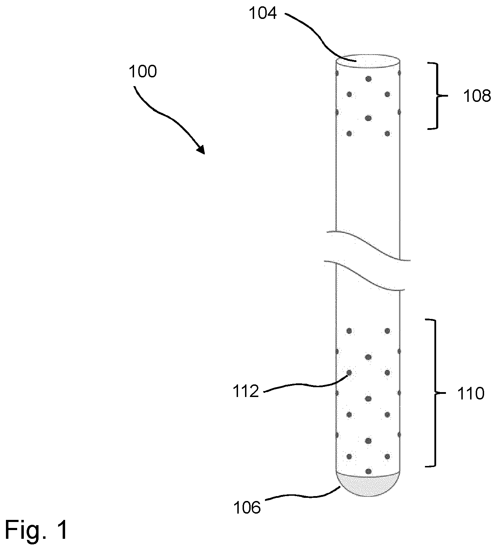

In **Fig. 1****,** a first embodiment of an elongated flexible electrode carrier 100, in particular for Deep Brain Stimulation, according to the present invention is shown, wherein the elongated flexible electrode carrier 100 has a proximal end 104 and a distal end 106.

Moreover, the elongated flexible electrode carrier 100 comprises a first zone 108 at the proximal end 104 and a second zone 110 at the distal end 106, wherein each of the zones 108, 110 comprises a plurality of electrodes 112.

Because the zones 108, 110 are spaced apart, two brain areas that are also spaced apart can be stimulated or recorded simultaneously or asynchronously.

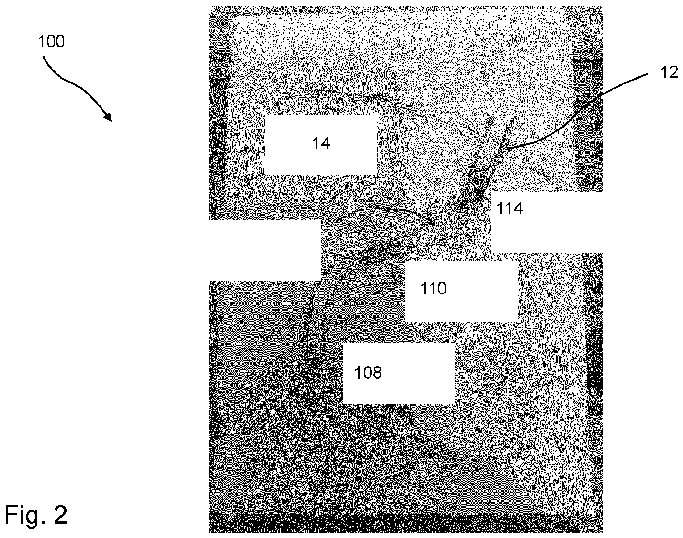

**Fig. 2** shows a second embodiment of an elongated flexible electrode carrier 100 inserted through opening 12 of the cranium 14.

The elongated flexible electrode carrier 100 comprises three zones 108, 110, 114.

Each of the zones 108, 110, and 114 comprises a plurality of electrodes 112.

The elongated flexible electrode carrier 100 in **Fig. 2** is bended as to adapt to three different brain areas (not shown) of the brain tissue. The flexibility enables the electrode to be adaptable, wherein the flexibility is preferably limited in such a way that the bended or flexed electrode carrier 100 substantially keeps its form.

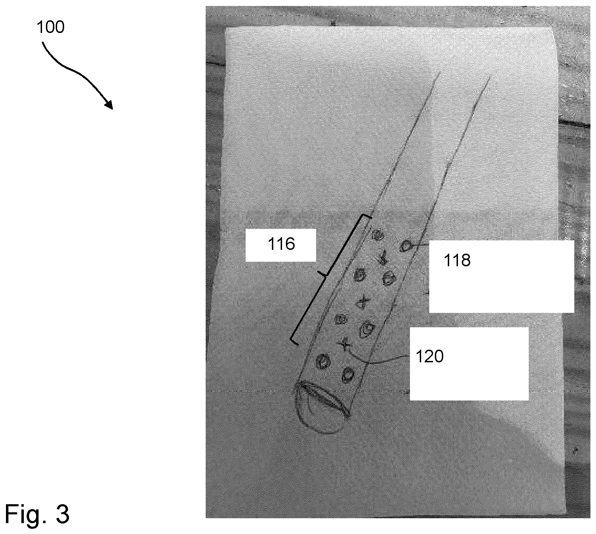

In **Fig. 3****,** an embodiment of a zone 116 at the distal end 106 of a flexible electrode carrier 100 is shown.

The zone 116 comprises a plurality of electrodes of a first type 118 being the default or general type used in combination with the flexible electrode carrier 100 as presented and a plurality of electrodes of second type 120 being specific electrodes which may be different to the first type electrode 118 due to their size, shape, material or the like. Both electrode types 118 ,120 are disposed in arrangements offset to each other or interlaced. Such arrangements can be (but are not limited) hexagonal arrangements, line arrangements, rectangular arrangements, symmetrical arrangements or asymmetrical arrangements. In particular, hexagonal arrangements are offering advantages in terms of symmetry and optimal mesh coverage.

Due to the pattern formed by the electrode types 118, 120 within the zone 116 shown in **Fig. 3****,** provided that both types 118, 120 are activated at the same time, the stimulation field is most likely a superimposed one. But it is also possible that e.g. the first type electrode 118 stimulates while the second type electrode 120 records the signals of the affected brain area.

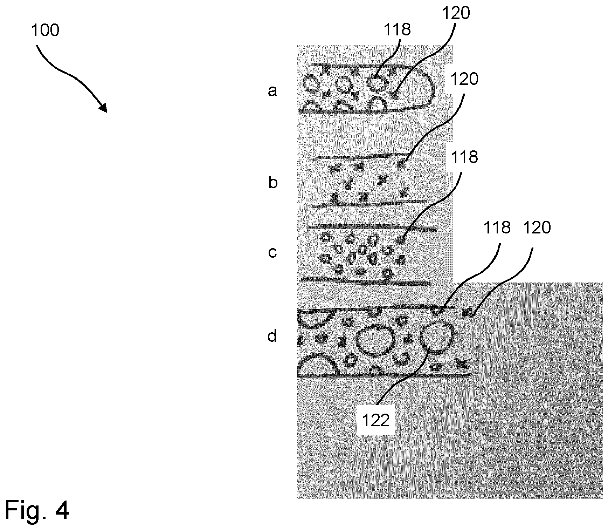

In **Fig. 4****,** different possible patterns within one zone of an elongated flexible electrode carrier 100 are presented. Pattern a corresponds to the pattern of electrode types 118, 120 shown in **Fig. 3****,** while pattern b and pattern c are the corresponding patterns with just one of the two electrode types 118, 120. Pattern d furthermore shows another electrode of a third type 122 which much greater than the first two electrode types 118, 120, whereby the pattern d results.

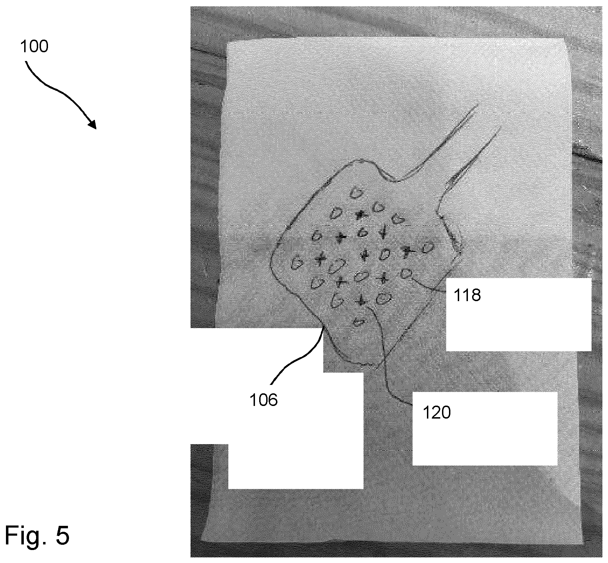

In **Fig. 5****,** an embodiment of a widened distal end 106 of a flexible electrode carrier 100 is shown, preferably used in Cortical Brain Stimulation, wherein the widened distal end 106 forms a kind of pad having a rectangular shape. However, other shapes matching the targeted brain area are possible as well.

Again, a plurality of first type electrodes 118 and second type electrodes 120 is arranged in lines offset to each other, but also other patterns are also possible. The electrodes 118, 120 are arranged in a plane to cover a corresponding area of the brain tissue to be stimulated.

The widened distal end 106 is flexible so that, on the one hand, the pad-like distal end 106 can adapt to the curvature of targeted brain area. On the other hand, the pad-like distal end 106 can be folded or wrapped around a longitudinal axis for the insertion of the flexible electrode carrier into a patient's cranium 14.

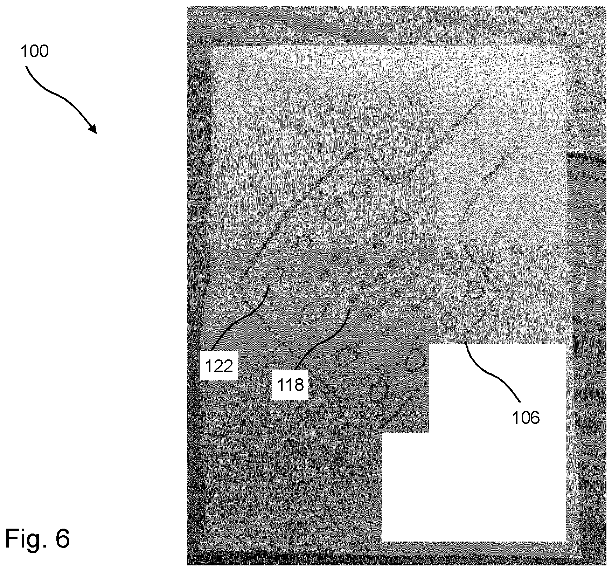

In **Fig. 6****,** another widened distal 106 is shown, wherein first type electrodes 118 are arranged inside third type electrodes 122 forming a frame.

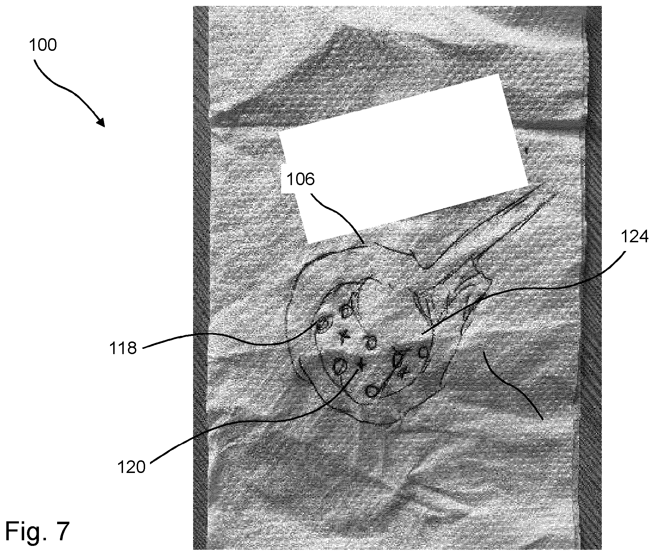

Finally in **Fig. 7****,** a further embodiment of a widened distal end 106 having first and second type electrode 118, 120 and forming a circular shape is shown. As presented in **Fig. 7****,** the widened distal end 106 may have at least one opening 124.

### Reference signs

- 100: flexible electrode carrier
- 102: antenna
- 104: proximal end
- 106: distal end
- 108: first zone
- 110: second zone
- 112: electrode
- 114: third zone
- 116: zone at distal end
- 118: first electrode type
- 120: second electrode type
- 122: third electrode type
- 200: wearable device
- 300: mobile device

## Claims

1. A flexible electrode carrier (100) for brain stimulation, in particular for Cortical Brain Stimulation and/or Deep Brain Stimulation, having a proximal end (104) and a distal end (106), wherein the flexible electrode carrier (100) comprises at least two zones (108, 110, 114, 116) each comprising a plurality of electrodes (112, 118, 120, 122);
wherein the material of which the electrodes (118, 120, 122) are made is reduced-graphene oxide,
wherein the plurality of electrodes (112, 118, 120, 122) in each of the at least two zones (108, 110, 114, 116) comprise at least one type of electrodes (118, 120, 122), and
wherein the types of electrodes (118, 120, 122) are each arranged in an individual pattern within the zones (108, 110, 114, 116).

2. The flexible electrode carrier (100) according to claim 1, wherein the flexible electrode carrier (100) is at least substantially elongated.

3. The flexible electrode carrier (100) according to claim 1 or 2, wherein the type of an electrode (118, 120, 122) is at least one of the group of size, shape, and material.

4. The flexible electrode carrier (100) according to one of the preceding claims, wherein the flexible electrode carrier (100) is widened at the distal end (106).

5. The flexible electrode carrier (100) according to one of the preceding claims, wherein the electrodes (112, 118, 120, 122) of one zone (108, 110, 114, 116) are embedded in a thin film substrate.

6. The flexible electrode carrier (100) according to one of the preceding claims, wherein said flexible electrode carrier (100) comprises at least 32 stimulation electrodes and at least 32 recording electrodes.

7. The flexible electrode carrier (100) according to one of the preceding claims, wherein electrodes (112, 118, 120, 122) of at least one zone (108, 110, 114, 116) are configured to generate a stimulation field, and electrodes (112, 118, 120, 122) of at least one other zone (108, 110, 114, 116) are configured to record brain signals.

8. The flexible electrode carrier (100) according to claim 7, wherein the recorded brain signals refer to an effect of the delivered stimulation.

9. The flexible electrode carrier (100) according to claim 1, wherein said plurality of electrodes (112, 118, 120, 122) comprises at least two different types of electrodes (112, 118, 120, 122),
wherein said at least two different types of electrodes (112, 118, 120, 122) are separately controlled.

10. The flexible electrode carrier (100) according to claim 1, wherein said plurality of electrodes (112, 118, 120, 122) comprises at least two different types of electrodes (112, 118, 120, 122),
wherein said at least two different types of electrodes (112, 118, 120, 122) are simultaneously controlled to generate a stimulation field.

11. The flexible electrode carrier (100) according to claim 10, wherein stimulation fields generated by the at least two types of electrodes (112, 118, 120, 122) are superimposing.

12. The flexible electrode carrier (100) according to claim 1, wherein said plurality of electrodes (112, 118, 120, 122) comprises at least two different types of electrodes (112, 118, 120, 122),
wherein at least a first type of electrodes (112, 118, 120, 122) is configured to generate a stimulation field and at least a second type of electrodes (112, 118, 120, 122), is configured to record emitted signals from an affected area of the brain,
wherein said different types of electrodes (112, 118, 120, 122), are individually connected and controlled.

13. The flexible electrode carrier (100) according to one of the preceding claims, wherein the at least two zones (108, 110, 114, 116) are spaced apart from each other.

14. The flexible electrode carrier (100) according to one of claims 1 to 12, wherein the at least two zones (108, 110, 114, 116) are adjacent to each other.

## Patentansprüche

1. Flexibler Elektrodenträger (100) für die Hirnstimulation, insbesondere für die kortikale Hirnstimulation und/oder die tiefe Hirnstimulation, mit einem proximalen Ende (104) und einem distalen Ende (106), wobei der flexible Elektrodenträger (100) mindestens zwei Bereiche (108, 110, 114, 116) aufweist, die jeweils eine Vielzahl von Elektroden (112, 118, 120, 122) umfassen;
wobei es sich bei dem Material, aus dem die Elektroden (118, 120, 122) bestehen, um reduziertes Graphenoxid handelt,
wobei die Vielzahl von Elektroden (112, 118, 120, 122) in jedem der mindestens zwei Bereiche (108, 110, 114, 116) mindestens eine Art von Elektroden (118, 120, 122) aufweist, und
wobei die Arten von Elektroden (118, 120, 122) innerhalb der Bereiche (108, 110, 114, 116) jeweils in einem individuellen Schema angeordnet sind.

2. Flexibler Elektrodenträger (100) nach Anspruch 1, wobei der flexible Elektrodenträger (100) zumindest im Wesentlichen langestreckt ist.

3. Flexibler Elektrodenträger (100) nach Anspruch 1 oder 2, wobei es sich bei der Art einer Elektrode (118, 120, 122) um mindestens ein Element aus der Gruppe bestehend aus Größe, Form und Material handelt.

4. Flexibler Elektrodenträger (100) nach einem der vorhergehenden Ansprüche, wobei der flexible Elektrodenträger (100) am distalen Ende (106) aufgeweitet ist.

5. Flexibler Elektrodenträger (100) nach einem der vorhergehenden Ansprüche, wobei die Elektroden (112, 118, 120, 122) eines Bereichs (108, 110, 114, 116) in ein Dünnschichtsubstrat eingebettet sind.

6. Flexibler Elektrodenträger (100) nach einem der vorhergehenden Ansprüche, wobei der flexible Elektrodenträger (100) mindestens 32 Stimulationselektroden und mindestens 32 Aufzeichnungselektroden aufweist.

7. Flexibler Elektrodenträger (100) nach einem der vorhergehenden Ansprüche, wobei Elektroden (112, 118, 120, 122) von mindestens einem Bereich (108, 110, 114, 116) dafür ausgelegt sind, ein Stimulationsfeld zu erzeugen, und Elektroden (112, 118, 120, 122) von mindestens einem anderen Bereich (108, 110, 114, 116) dafür ausgelegt sind, Hirnsignale aufzuzeichnen.

8. Flexibler Elektrodenträger (100) nach Anspruch 7, wobei sich die aufgezeichneten Hirnsignale auf die Wirkung der verabreichten Stimulation beziehen.

9. Flexibler Elektrodenträger (100) nach Anspruch 1, wobei die Vielzahl von Elektroden (112, 118, 120, 122) mindestens zwei verschiedene Arten von Elektroden (112, 118, 120, 122) umfasst,
wobei die mindestens zwei verschiedenen Arten von Elektroden (112, 118, 120, 122) separat gesteuert werden.

10. Flexibler Elektrodenträger (100) nach Anspruch 1, wobei die Vielzahl von Elektroden (112, 118, 120, 122) mindestens zwei verschiedene Arten von Elektroden (112, 118, 120, 122) umfasst,
wobei die mindestens zwei verschiedenen Arten von Elektroden (112, 118, 120, 122) gleichzeitig gesteuert werden, um ein Stimulationsfeld zu erzeugen.

11. Flexibler Elektrodenträger (100) nach Anspruch 10, wobei sich Stimulationsfelder, die von den mindestens zwei Arten von Elektroden (112, 118, 120, 122) erzeugt werden, einander überlagern.

12. Flexibler Elektrodenträger (100) nach Anspruch 1, wobei die Vielzahl von Elektroden (112, 118, 120, 122) mindestens zwei verschiedene Arten von Elektroden (112, 118, 120, 122) umfasst,
wobei mindestens eine erste Art von Elektroden (112, 118, 120, 122) dafür ausgelegt ist, ein Stimulationsfeld zu erzeugen, und mindestens eine zweite Art von Elektroden (112, 118, 120, 122) dafür ausgelegt ist, von einem betroffenen Hirnareal ausgesendete Signale aufzuzeichnen,
wobei die verschiedene Arten von Elektroden (112, 118, 120, 122) einzeln angeschlossen sind und gesteuert werden.

13. Flexibler Elektrodenträger (100) nach einem der vorhergehenden Ansprüche, wobei die mindestens zwei Bereiche (108, 110, 114, 116) voneinander beabstandet sind.

14. Flexibler Elektrodenträger (100) nach einem der Ansprüche 1 bis 12, wobei die mindestens zwei Bereiche (108, 110, 114, 116) aneinander angrenzen.

## Revendications

1. Support d'électrode flexible (100) pour une stimulation cérébrale, notamment pour une stimulation cérébrale corticale et/ou une stimulation cérébrale profonde, ayant une extrémité proximale (104) et une extrémité distale (106), dans lequel le support d'électrode flexible (100) comprend au moins deux zones (108, 110, 114, 116) comprenant chacune une pluralité d'électrodes (112, 118, 120, 122) ;
dans lequel le matériau à partir duquel les électrodes (118, 120, 122) sont fabriquées est un oxyde de graphène réduit ;
dans lequel la pluralité d'électrodes (112, 118, 120, 122) dans chacune des au moins deux zones (108, 110, 114, 116) comprennent au moins un type d'électrodes (118, 120, 122) ; et
dans lequel les types d'électrodes (118, 120, 122) sont chacune agencées dans un motif individuel à l'intérieur des zones (108, 110, 114, 116).

2. Support d'électrode flexible (100) selon la revendication 1, dans lequel le support d'électrode flexible (100) est au moins sensiblement allongé.

3. Support d'électrode flexible (100) selon la revendication 1 ou 2, dans lequel le type d'électrode (118, 120, 122) est au moins un élément dans le groupe composé de la taille, de la forme et du matériau.

4. Support d'électrode flexible (100) selon l'une quelconque des revendications précédentes, dans lequel le support d'électrode flexible (100) est élargi à l'extrémité distale (106).

5. Support d'électrode flexible (100) selon l'une quelconque des revendications précédentes, dans lequel les électrodes (112, 118, 120, 122) d'une zone (108, 110, 114, 116) sont encastrées dans un substrat de film fin.

6. Support d'électrode flexible (100) selon l'une quelconque des revendications précédentes, dans lequel ledit support d'électrode flexible (100) comprend au moins 32 électrodes de stimulation et au moins 32 électrodes d'enregistrement.

7. Support d'électrode flexible (100) selon l'une quelconque des revendications précédentes, dans lequel les électrodes (112, 118, 120, 122) d'au moins une zone (108, 110, 114, 116) sont configurées pour générer un champ de stimulation et dans lequel les électrodes (112, 118, 120, 122) d'au moins une autre zone (108, 110, 114, 116) sont configurées pour enregistrer les signaux cérébraux.

8. Support d'électrode flexible (100) selon la revendication 7, dans lequel les signaux cérébraux enregistrés font référence à un effet de la stimulation délivrée.

9. Support d'électrode flexible (100) selon la revendication 1, dans lequel ladite pluralité d'électrodes (112, 118, 120, 122) comprend au moins deux types d'électrodes (112, 118, 120, 122) différents,
dans lequel lesdits au moins deux types d'électrodes (112, 118, 120, 122) différents sont commandés séparément.

10. Support d'électrode flexible (100) selon la revendication 1, dans lequel ladite pluralité d'électrodes (112, 118, 120, 122) comprend au moins deux types d'électrodes (112, 118, 120, 122) différents ;
dans lequel lesdits au moins deux types d'électrodes (112, 118, 120, 122) différents sont commandés simultanément pour générer un champ de stimulation.

11. Support d'électrode flexible (100) selon la revendication 10, dans lequel les champs de stimulation générés par les au moins deux types d'électrodes (112, 118, 120, 122) sont superposés.

12. Support d'électrode flexible (100) selon la revendication 1, dans lequel ladite pluralité d'électrodes (112, 118, 120, 122) comprend au moins deux types d'électrodes (112, 118, 120, 122) différents ;
dans lequel au moins un premier type d'électrodes (112, 118, 120, 122) est configuré pour générer un champ de stimulation et au moins un second type d'électrodes (112, 118, 120, 122), est configuré pour enregistrer les signaux émis depuis une zone affectée du cerveau ;
dans lequel lesdits différents types d'électrodes (112, 118, 120, 122) sont reliés et commandés de façon individuelle.

13. Support d'électrode flexible (100) selon l'une quelconque des revendications précédentes, dans lequel les au moins deux zones (108, 110, 114, 116) sont séparées l'une de l'autre.

14. Support d'électrode flexible (100) selon l'une quelconque des revendications 1 à 12, dans lequel les au moins deux zones (108, 110, 114, 116) sont côte à côte.
